(19) Europäisches Patentamt
European Patent Office
Office européen des brevets

(11) Numéro de publication: **0 000 301**
**B1**

(12) **FASCICULE DE BREVET EUROPEEN**

(21) Numéro de dépôt: **78400018.4**

(22) Date de dépôt: **13.06.78**

(51) Int. Cl.³: **C 07 D 495/04**
// (C 07 D 495/04, 333/00, 221/00)

(54) **Procédé de préparation de thiéno(2,3-c) et thiéno(3,2-c) pyridines**

(30) Priorité: **21.06.77 FR 7718991**

(43) Date de publication de la demande:
**10.01.79 Bulletin 79/01**

(45) Mention de la délivrance du brevet:
**17.09.80 Bulletin 80/19**

(84) Etats contractants désignés:
**DE NL SE**

(56) Documents cités:
**FR - A - 2 312 498**
**BULLETIN OF THE CHEMICAL SOCIETY OF JAPAN, 47, 1297—98 (1974) Page 1297, colonne 1, lignes 21—40**

(73) Titulaire: **OMNIUM FINANCIER AQUITAINE POUR L'HYGIENE ET LA SANTE—SANOFI**
**Tour Aquitaine Cedex No 4**
**F-92080 Paris La Defense (FR)**

(72) Inventeur: **Maffrand, Jean-Pierre**
**10, Rue Jean Mermoz**
**F-31300 Toulouse (FR)**

(74) Mandataire: **Lavoix, Jean**
**c/o Cabinet Lavoix 2, Place D'Estienne D'Orves**
**F-75441 Paris Cedex 09 (FR)**

(56) Documents cités:
**FR - A - 2 312 498**
**BULLETIN OF THE CHEMICAL SOCIETY OF JAPAN, 47, 1297—98 (1974) Page 1297, colonne 1, lignes 21—40**

**EP 0 000 301 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de thiéno[2,3-c] et thiéno[3,2-c]pyridines

La présente invention est relative à un nouveau procédé de préparation de thiéno[2,3-c] et thiéno[3,2-c]pyridines de formules:

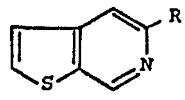

ou

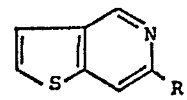

I  II

[2,3-c]  [3,2-c]

dans lesquelles R représente l'hydrogène ou le groupe carboxy. Plusieurs méthodes conduisant aux dérivés de formules I et II (R = H) ont été décrites dans la littérature, mais elles sont difficilement transposables à l'échelle industrielle et/ou sont trop onéreuses. Ainsi, les voies d'accès mentionnées par W. HERTZ et L. TSAI (J. Amer. Chem. Soc., 1953, *75*, 5122) ou par C. HANSCH, W. CARPENTER et J. TODD (J. Org. Chem. 1958, *23*, 1924) ou par L. H. KLEMM, J. SHABTOY, D. R. McCOY et W. K. KRIANG (J. Het. Chem., 1968, 5883 et ibid. 1969, 6813) ou par S. GRONOWITZ et E. SANDBERG (Ark. Kemi., 1970, *32*, 217) présentent les deux inconvénients cités plus haut.

Par ailleurs, la méthode de F. ELOY et A. DERYCKERE (Bull. Soc. Chim. Belges, 1970, *79*, 301) fait intervenir un azide qui présente des risques d'explosion. Enfin, les procédés décrits par J. P. MAFFRAND et F. ELOY (J. Het. Chem., 1976, *13*,, 1347) et par A. HEYMES et J. P. MAFFRAND (Demande de brevet français publiée N° 2 312 498) sont plus coûteux que celui de la présente invention.

Les dérivés de formules (I) et (II) dans lesquelles R = COOH n'ont été décrits qu'une fois dans la littérature par M. FARNIER, S. SOTH et P. FOURNARI (Can. J. Chem., 1976, *54*, 1067), mais le procédé utilisé pour les préparer ne permet pas d'obtenir ces produits en grandes quantités.

La présente invention a pour but de fournir un procédé de synthèse peu onéreux permettant d'obtenir, avec de bons rendements, les composés (I) et (II) qui sont des intermédiaires importants dans l'industrie chimique et pharmaceutique, en particulier pour la préparation de dérivés de thiéno-pyridines présentant diverses activités thérapeutiques, par exemple anti-inflammatoire, anti-agrégation plaquettaire, anti-arythmique, etc... (voir par exemple les brevets et demandes de brevet français publiées N° 2 215 948, 2 257 271, 2 315 274 et 2 345 150.

Le procédé de l'invention est caractérisé en ce qu'on fait réagir un composé de formule

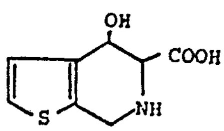

ou

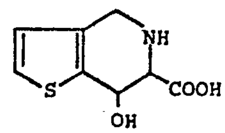

(III)  (IV)

avec de l'acide nitreux, obtenant ainsi des composés de formule

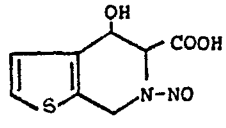

ou

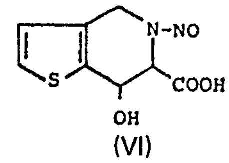

(V)  (VI)

et on fait réagir les composés de formule (V) ou (VI) respectivement soit avec un acide, obtenant ainsi les dérivés de formule (I) et (II) respectivement dans lesquels R est l'hydrogène, soit avec un hydroxyde de métal alcalin et neutralisation subséquent, obtenant ainsi les dérivés de formule (I) et (II) respectivement dans lesquels R est le groupe carboxy.

On forme de préférence l'acide nitreux *in situ* par réaction d'un nitrite de métal alcalin en solution aqueuse avec un acide.

La réaction est effectuée notamment en ajoutant lentement une solution aqueuse de nitrite de métal alcalin, notamment de sodium, à une solution chlorhydrique, maintenue à 0°—15°C, du dérivé

de formule (III) ou (IV) puis en abandonnant plusieurs heures à température ambiante.

Le traitement de la nitrosoamine de formule (V) ou (VI) est effectué à l'aide d'un acide minéral tel que l'acide chlorhydrique, l'acide bromhydrique ou l'acide sulfurique, de préférence l'acide chlorhydrique, ou d'un acide organique, tel que l'acide trifluoroacétique ou l'acide trichloroacétique, de préférence l'acide trifluoroacétique, pour aboutir à la thiénopyridine de formule (Ia) ou (IIa) dans laquelle R=H.

Avec l'acide trifluoroacétique pur, la réaction est très exothermique, alors qu'il est nécessaire de chauffer avec l'acide chlorhydrique pour réaliser la transformation.

Le chauffage à reflux des mêmes dérivés de formule (III) ou (IV) dans une solution aqueuse d'un hydroxyde de métal alcalin, la soude de préférence conduit après neutralisation respectivement aux acides de formule (Ib) ou (IIb) (R = COOH). Si on le désire, ces acides peuvent d'ailleurs être décarboxylés au moyen de poudre de cuivre en présence de quinoléine, selon M. FARNIER, S. SOTH et P. FOURNARI (Can. J. Chem. 1976, *54*, 1067) pour donner les composés de formule (Ia) ou (IIa) (R = H).

Ce procédé peut être représenté par le schéma réactionnel suivant:

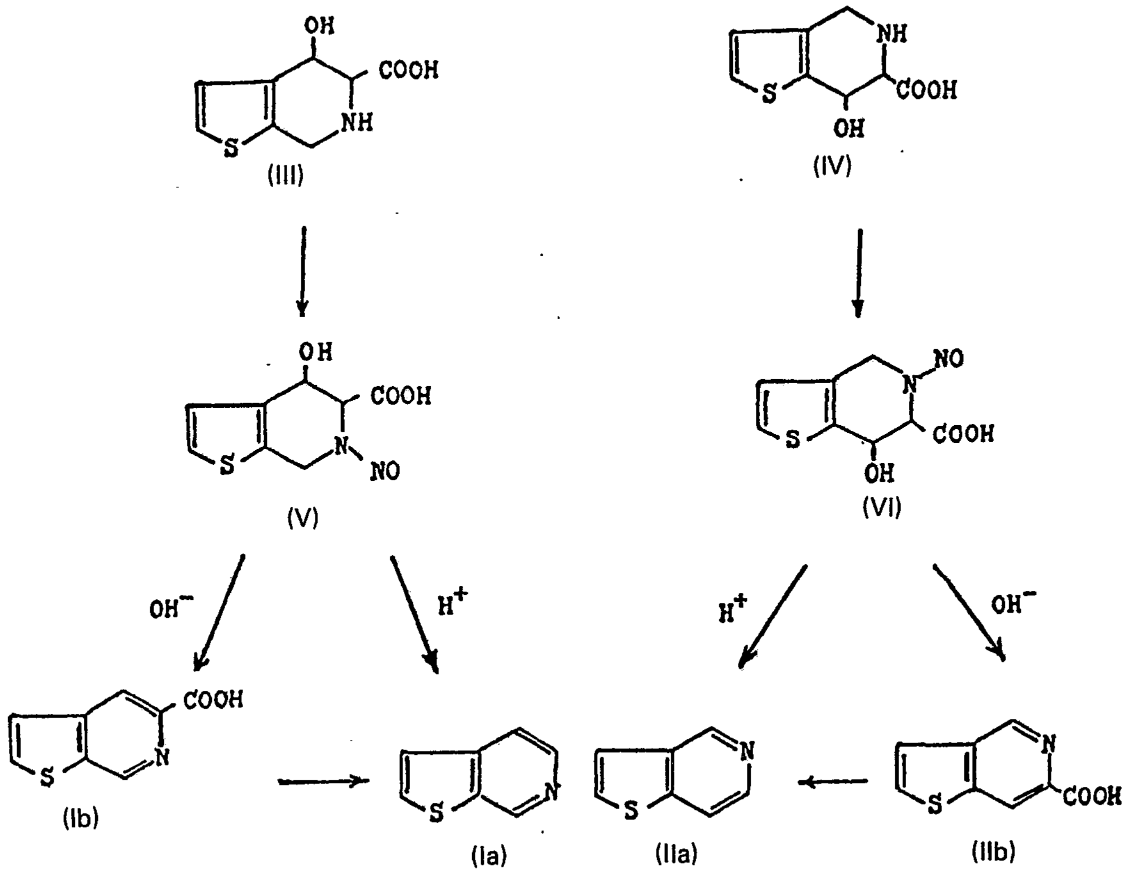

(III) (IV) (V) (VI) (Ib) (Ia) (IIa) (IIb)

Les composés de départ de formule (III) ou (IV) peuvent être préparés par réaction d'un composé de formule

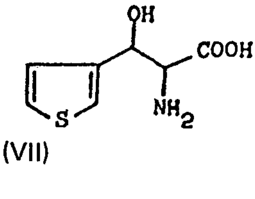
(VII)

ou

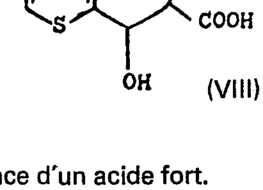
(VIII)

avec du formaldéhyde en solution aqueuse, en présence d'un acide fort.

Les sérines de formule (VII) ou (VIII) peuvent être obtenues comme suit:

—la β-(thiényl-2) sérine peut être préparée selon G. Weitnauer, Gazz. Chim. Ital. 1951, 81, 162

—la β-(thiényl-3) sérine peut être préparée à partir du thiénaldéhyde-3 en adoptant le procédé de Weitnauer ci-dessus — chlorhydrate cristaux blancs, F = 241 °C.

Les exemples non limitatifs suivants sont donnés à titre d'illustration de l'invention.

Exemple 1.

Préparation de la carboxy-5 hydroxy-4 nitroso-6 tétrahydro-4,5,6,7 thiéno[2,3-c]pyridine

A une suspension agitée magnétiquement et maintenue à 10°C de 20 g (0,1 mole) de carboxy-5 hydroxy-4 tétrahydro-4,5,6,7 thiéno-[2,3-c]pyridine dans 200 $cm^3$ d'acide chlorhydrique 3N, on ajoute, goutte à goutte, 200 $cm^3$ d'une solution aqueuse à 10% de nitrite de sodium (2,9 équivalents) et on agite à température ambiante pendant 3 heures. Le milieu reste hétérogène pendant toute la durée de l'opération et on observe un dégagement de vapeurs nitreuses. Le précipité obtenu est filtré, lavé à l'eau et séché sous vide: Cristaux beiges, fusion pâteuse à partir de 100°C (21,3 g, 93%).

Exemple 2.

En opérant comme à l'exemple 1, on obtient la carboxy-6 hydroxy-7 nitroso-5 tétrahydro-4,5,6,7 thiéno[3,2-c]pyridine. Fusion pâteuse à partir de 60°C, rendement: 97%.

Exemple 3.

Préparation de la thiéno[3,2-c]pyridine

On chauffe à 60°C pendant 2 heures une solution de 24 g du dérivé nitrosé obtenu à l'Exemple 2 dans 200 $cm^3$ d'acide chlorhydrique 6N. On observe un dégagement gazeux et la formation de vapeurs rousses. Après refroidissement, le milieu réactionnel brun est basifié avec de la lessive de soude et extrait au chlorure de méthylène. Les extraits organiques sont lavés à l'eau, séchés sur sulfate de sodium, décolorés au noir, filtrés sur talc, et évaporés à sec. La distillation sous vide du résidu fournit 6,5 g (rendement global à partir du produit de départ de formule (IV); 43%) de thiéno[3,2-c]pyridine qui cristallise au refroidissement. F $<$ 50°C.

Exemple 4.

En opérant comme à l'exemple 3 en partant du composé nitrosé obtenu à l'Exemple 1, on obtient la thiéno[2,3-c]pyridine. F $<$ 50°C, rendement: 47%.

Exemple 5.

Préparation de la carboxy-5 thiéno[2,3-c]pyridine

On chauffe à reflux pendant 2 heures une solution initialement homogène de 10 g (0,044 mole) du dérivé nitrosé obtenu à l'exemple 1, 20 $cm^3$ d'éthanol et 60 $cm^3$ d'hydroxyde de sodium à 20%. Après refroidissement et addition d'éthanol, le précipité obtenu est filtré, lavé à l'éthanol puis à l'éther et séché. Le sel sodique obtenu (F = 260°, 4,7 g, 60%) est traité par 23 $cm^3$ (1 équivalent) d'acide chlorhydrique N. On observe une dissolution puis une reprécipitation. On recristallise directement après avoir ajouté 27 $cm^3$ d'eau. On obtient 2,5 g (32%) de cristaux rosés, F = 246°C.

Exemple 6.

En opérant comme à l'Exemple 5 en partant du dérivé nitrosé de l'Exemple 2, on obtient la carboxy-6 thiéno[3,2-c]pyridine. Cristaux rosés, F = 212°C, rendement: 84%.

Exemple 7.

Préparation de la thiéno[3,2-c]pyridine

On ajoute, par portions, 11,4 g du dérivé de formule VI de l'exemple 2 à 55 $cm^3$ d'acide trifluoroacétique agités à température ambiante. La température passe de 19°C à 34°C et il y a dégagement de vapeurs rousses. On laisse revenir à température ambiante, verse le mélange réactionnel sur de la glace, basifie par addition d'ammoniaque concentrée et extrait à l'éther diisopropylique. Les extraits organiques sont lavés à l'eau, séchés sur sulfate de sodium et évaporés à sec. La distillation sous vide du résidu fournit 3,8 g (rendement 56%) de thiéno[3,2-c]pyridine.

**Revendications**

1. Procédé de préparation de dérivés de formule

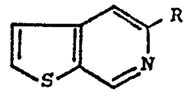

ou

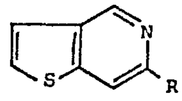

I [2,3-c]

II [3,2-c]

dans lesquelles R représente l'hydrogène ou le groupe carboxy, caractérisé en ce qu'on fait réagir un composé de formule

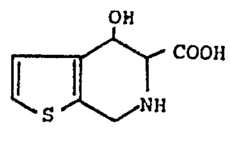

ou

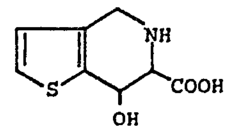

(III) (IV)

avec de l'acide nitreux, obtenant ainsi respectivement des composés de formules

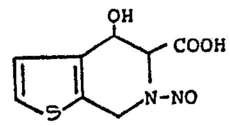

et

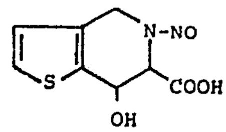

(V) (VI)

et on fait réagir les composés de formules (V) et (VI) respectivement soit avec un acide, obtenant ainsi les dérivés de formules (I) et (II) respectivement dans lesquels R est l'hydrogène, soit avec un hydroxyde de métal alcalin et neutralisation subséquente, obtenant ainsi les dérivés de formules (I) et (II) respectivement dans lesquels R est le groupe carboxy.

2. Procédé suivant la revendication 1, caractérisé en ce qu'on forme l'acide nitreux *in situ* par réaction d'un nitrite de métal alcalin en solution aqueuse avec un acide.

3. Procédé suivant la revendication 2, caractérisé en ce que l'acide est l'acide chlorhydrique.

4. Procédé suivant la revendication 1, caractérisé en ce que la dénitrosation par l'acide est effectuée à l'aide d'un acide minéral ou organique.

5. Procédé suivant la revendication 4, caractérisé en ce que l'acide minéral est l'acid chlorhydrique.

6. Procédé suivant la revendication 4, caractérisé en ce que l'acide organique est l'acide trifluoroacétique.

7. Procédé suivant la revendication 1, caractérisé en ce que la dénitrosation par l'hydroxyde de métal alcalin est effectuée en solution aqueuse à la température de reflux.

8. Procédé suivant la revendication 1, caractérisé en ce qu'on décarboxyle un composé de formule (I) ou (II) dans lequel R est le groupe carboxy pour obtenir le composé de formule (I) ou (II) correspondant dans lequel R est l'hydrogène.

9. Procédé suivant la revendication 8, caractérisé en ce qu'on effectue la décarboxylation au moyen de poudre de cuivre en présence de quinoléine.

**Claims**

1. Process for the preparation of derivatives having the formula

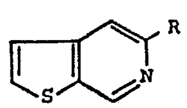

or

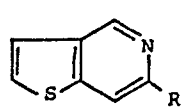

(I) (II)

[2,3-c] [3,2-c]

wherein R is hydrogen or carboxy, characterized in that a compound of the formula

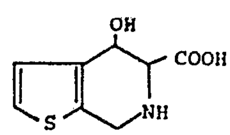

or

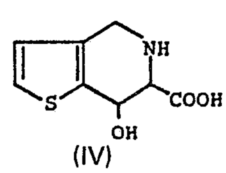

(III) (IV)

is reacted with nitrous acid, to give compounds of the formula

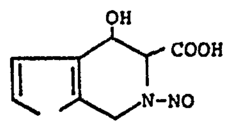

or

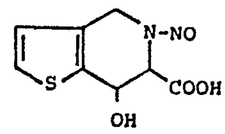

(V) (VI)

and the compounds of the formula (V) or (VI) are reacted either with an acid, to give the derivatives of the formulae (I) and (II), respectively, in which R is hydrogen, or with an alkali metal hydroxide with subsequent neutralization, to give the derivatives of the formulae (I) and (II), respectively, in which R is the carboxy group.

2. Process as claimed in claim 1, wherein the nitrous acid is formed *in situ*, by reaction of an aqueous solution of an alkali metal nitrite with an acid.

3. Process as claimed in claim 2, wherein said acid is hydrochloric acid.

4. Process as claimed in claim 1, wherein said denitrosation with the acid is effected with an inorganic or organic acid.

5. Process as claimed in claim 4, wherein said inorganic acid is hydrochloric acid.

6. Process as claimed in claim 4, wherein said organic acid is trifluoroacetic acid.

7. Process as claimed in claim 1, wherein said denitrosation with said alkali metal hydroxide is effected in aqueous solution at the reflux temperature.

8. Process as claimed in claim 1, wherein a compound selected from the compounds of the formulae (I) and (II) in which R is carboxy is decarboxylated to give the corresponding compound of the formula (I) or (II) in which R is hydrogen.

9. Process as claimed in claim 8, wherein said decarboxylation is effected with copper powder in the presence of quinoline.

**Patentansprüche**

1. Verfahren zur Herstellung von Thieno[2,3-c]- und Thieno[3,2-c]pyridinderivaten der allgemeinen Formel

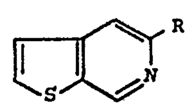

oder

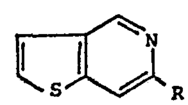

(I) (II)

worin R Wasserstoff oder Carboxyl bedeutet, dadurch gekennzeichnet, dass man eine Verbindung der Formel

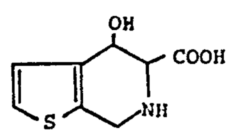

oder

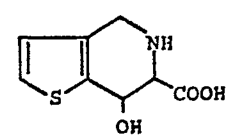

(III) (IV)

mit salpetriger Säure umsetzt und die so erhaltenen Verbindungen der Formel

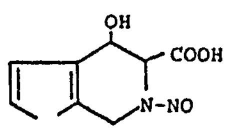

bzw.

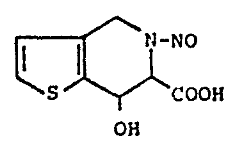

(V) (VI)

entweder mit einer Säure, wobei die Verbindungen (I) bzw. (II), worin R Wasserstoff bedeutet, erhalten werden, oder mit einem Alkalimetallhydroxyd und anschliessende Neutralisation, wobei die Verbindungen (i) bzw. (II), worin R Carboxyl bedeutet, erhalten werden, umsetzt.

2. Verfahren nach Anspruch 1, dadurch gekennzeichnet, dass man die salpetrige Säure in situ durch Umsetzen eines Alkalimetallnitrits in wässeriger Lösung mit einer Säure bildet.

3. Verfahren nach Anspruch 2, dadurch gekennzeichnet, dass man als Säure Salzsäure einsetzt.

4. Verfahren nach Anspruch 1, dadurch gekennzeichnet, dass man die Entfernung der Nitrosogruppe mittels einer Säure mit einer Mineralsäure oder einer organischen Säure bewirkt.

5. Verfahren nach Anspruch 4, dadurch gekennzeichnet, dass man als Mineralsäure Salzsäure einsetzt.

6. Verfahren nach Anspruch 4, dadurch gekennzeichnet, dass man als organische Säure Trifluoressigsäure einsetzt.

7. Verfahren nach Anspruch 1, dadurch gekennzeichnet, dass man die Entfernung der Nitrosogruppe mittels eines Alkalimetallhydroxyds in wässeriger Lösung bei Rückflusstemperatur bewirkt.

8. Verfahren nach Anspruch 1, dadurch gekennzeichnet, dass man eine Verbindung der Formel (I) oder (II), worin R Carboxyl bedeutet, zu einer Verbindung (I) oder (II), worin R Wasserstoff ist, decarboxyliert.

9. Verfahren nach Anspruch 8, dadurch gekennzeichnet, dass man die Decarboxylierung mit Hilfe von Kupferpulver in Anwesenheit von Chinolin durchführt.